# EUROPÄISCHE PATENTSCHRIFT

(11) **EP 2 078 503 B1**
(45) Veröffentlichungstag und Bekanntmachung des Hinweises auf die Patenterteilung: **23.02.2011**
(21) Anmeldenummer: 07024516.2
(22) Anmeldetag: 18.12.2007
(51) Int. Cl.: A61B 17/22, A61B 19/00

(54) **Navigation bei der fokussierten Druckwellenbehandlung**
Navigation for focussed pressure wave treatment
Navigation pour le traitement par onde de pression focalisée

(43) Veröffentlichungstag der Anmeldung: 15.07.2009
(73) Patentinhaber: STORZ MEDICAL AG, 8274 Tägerwilen (CH)
(72) Erfinder: Hagelauer, Ulrich, 78464 Konstanz (DE)
(74) Vertreter: Szynka, Dirk

(56) Entgegenhaltungen:
- EP-A- 0 382 392
- WO-A-01/03589
- DE-A1- 19 622 920
- FR-A- 2 686 499

## Beschreibung

Die vorliegende Erfindung betrifft das Gebiet der fokussierten Druckwellenbehandlung des menschlichen und tierischen Körpers und die Navigation in diesem Bereich.

Druckwellen, d. h. mechanische und gelegentlich auch als "akustisch" bezeichnete Wellen werden in verschiedener Weise zur therapeutischen Behandlung eingesetzt. Besonders wichtig und historisch betrachtet auch Ausgangspunkt der Entwicklung ist die Stoßwellenlithotripsie, also die Zerkleinerung von Körperkonkrementen, insbesondere Steinen, mit fokussierten Druckwellen großer Amplituden und mit steilen Anstiegsflanken. Hier werden einzelne Pulse auf das Konkrement gerichtet, deren erste, einer Kompression entsprechende "Halbwelle" hinsichtlich Flankensteilheit und Amplitude sowie therapeutischer Wirkung dominiert, wohingegen bereits die nächste nachfolgende Halbwelle, die einer Expansion entspricht, deutlich weniger ausgeprägt ist. Solche Pulse werden regelmäßig wiederholt angewendet.

Vergleichbare Verfahren mit Stoßwellen sind auch für andere Indikationen bekannt, etwa zur Beaufschlagung von schlecht heilenden Knochenbrüchen.

Daneben betrifft die Erfindung aber auch Druckwellentherapien mit eigentlichen, also fortgesetzt oszillierenden Wellen. Diese können in fokussierter Weise zur Erwärmung von Körpergewebe eingesetzt werden, etwa zur sog. thermischen Ablation von Tumoren.

Die wesentlichen Frequenzen liegen bei den genannten Therapien über der Hörschwelle; es handelt sich also um Ultraschallverfahren.

Obwohl auch Therapien mit nicht fokussierten Druckwellen bekannt sind, richtet sich die vorliegende Erfindung auf Anwendungen mit fokussierten Wellen (wobei Pulse inbegriffen sein sollen, vgl. oben). Wenngleich die Abgrenzung zwischen fokussierten und nicht fokussierten Wellen Schwierigkeiten bereiten kann, soll es im Folgenden jedenfalls nur um solche Therapien gehen, bei denen die Druckwellen bewusst auf eine mehr oder weniger ausgedehnte Körperregion konzentriert werden, um erhöhte Intensitäten, Drücke oder Flankensteilheiten zu erzielen.

Da bei diesen fokussierenden Therapien die Lokalisierung auf die zu behandelnde Körperregion naturgemäß wesentlich ist, spielt die Einstellung der entsprechenden Vorrichtungen zur richtigen Anordnung des Fokusbereichs im Körper eine entscheidende Rolle. Dies betrifft natürlich zum einen die anfängliche Einstellung auf die zu behandelnde Region, etwa einen Stein. Wenn hier zu große Toleranzen auftreten, wird gesundes Gewebe beschädigt oder unnötig viel gesundes Gewebe in Mitleidenschaft gezogen und andererseits der Therapieerfolg in der eigentlich zu behandelnden Region eingeschränkt oder gefährdet. Man spricht hier von der "Navigation".

Erschwerend kommt hinzu, dass die Navigation kein statischer Vorgang sein muss, also Änderungen während der Behandlung auftreten können. Eine wesentliche Ursache sind Bewegungen des Patienten oder Verschiebungen von Organen, insbesondere infolge seiner Atmung.

Zur Navigation können bildgebende Verfahren herangezogen werden, die die zu behandelnde Region von der Umgebung unterscheidbar machen und Navigationsinformationen dazu, also letztlich Koordinaten, für die Druckwellenvorrichtung zur Verfügung stellen können. Bekannt ist insbesondere die laufende Röntgenüberwachung während der Stoßwellenlithotripsie. Da mindestens zwei Röntgenprojektionen für die Festlegung der räumlichen Position erforderlich sind, entsteht ein erheblicher technischer Aufwand für das Verschwenken der Röntgenachsen und entsprechende Kosten. Für den Patienten verursacht die Röntgenüberwachung eine Strahlenbelastung.

Das Dokument FR 2 686 499 A beschreibt das Einbringen von Markern vor einer Stoßwellenbehandlung. Dabei sollen komplexe und teure, aber sehr gut auflösende Bildgebungsverfahren zur Markerpositionierung ausgenutzt werden, die im Umfeld der Stoßwellenbehandlung nicht verfügbar sind.

Das Dokument EP 0 382 392 A auf dem der Oberbegriff von Anspruch 1 beruht, beschreibt die Einführung eines luftgefüllten Ballonkatheters und dessen Positionierung in der Nähe eines mit Stoßwellen zu zertrümmernden Steins, und zwar zum Zweck der Ultraschallortung.

Das Dokument WO 01/03589 A befasst sich mit der Abtragung von Plaque in Blutgefäßen durch eine an der Spitze eines Katheters angebrachte Energiequelle, die auch magnetisch manipuliert werden und dazu aus magnetischem Material bestehen kann.

Der vorliegenden Erfindung liegt davon ausgehend das Problem zugrunde, eine hinsichtlich der Navigation verbesserte Vorrichtung zur Behandlung des menschlichen oder tierischen Körpers mit fokussierten Druckwellen anzugeben.

Dieses Problem wird gelöst, durch eine Vorrichtung gemäß Anspruch 1.

Die Grundidee der Erfindung liegt darin, die Kombination einer Ortungssonde und eines Magnetortungssystems einzusetzen. Die Ortungssonde ist ein minimalinvasives Instrument zum Einführen in den Körper, und zwar in die Nähe, wenn auch nicht notwendigerweise in die unmittelbare Nähe, der zu behandelnden Region. Es kann sich hierbei insbesondere um einen Katheter oder ein Endoskop handeln, wobei die begriffliche Unterscheidung bedeuten soll, dass Katheter ohne optische Sichteinrichtung und Endoskope mit optischer Sichteinrichtung gemeint sind. Das Endoskop könnte also auch flexibel sein und der Katheter, jedenfalls prinzipiell, auch starr.

Magnetortungssysteme sind an sich bekannt und werden häufig als Tracking-Systeme bezeichnet. Im Körper wird ein Ortungselement angeordnet, das eine aktive oder passive Spule oder auch ein Dauermagnet sein könnte. Dieses Ortungselement kann von einem extrakorporalen Magnetortungsgerät geortet und seine Lage koordinatenmäßig bestimmt werden. Das Magnetortungssystem dient zur Navigation der erfindungsgemäßen Druckwellenvorrichtung; die Druckwellenvorrichtung ist also auf die Koordinatenangaben des Magnetortungssystems "geeicht", d. h. referenziert. Das Magnetortungselement ist erfindungsgemäß in der Ortungssonde angeordnet, vorzugsweise in deren Spitze oder in der Nähe der Spitze. Es wird also ebenfalls in die Nähe der zu behandelnden Region gebracht. Dann kann in verschiedenster Weise der Abstand zwischen dem Magnetortungselement und der zu behandelnden Region festgelegt werden. Dies beinhaltet sowohl eine Messung des Abstandes, wenn das Magnetortungselement nicht in der unmittelbaren Umgebung bzw. nicht in Kontakt mit der zu behandelnden Region angeordnet ist, als auch eine Minimierung des Abstandes auf ein therapeutisch unwesentliches Maß, d. h. eine unmittelbare Anordnung des Magnetortungselements an der zu behandelnden Region. Der Maßstab für die Unwesentlichkeit des minimierten Abstandes ist dabei der Fokusbereich der Druckwellen. Man geht hier also davon aus, dass bei einer ausreichend nahen Anordnung des Magnetortungselementes an der zu behandelnden Region der verbleibende Positionsunterschied druckwellentherapeutisch keine wesentliche Rolle mehr spielt und somit die Position des Magnetortungselements bei der Navigation als "Ziel" verwendet werden kann. Anderenfalls wird, wie bereits erläutert, der verbleibende Abstand gemessen und rechnerisch berücksichtigt.

Das Magnetortungssystem bietet eine kostengünstige und für den Patienten belastungsfreie Navigationstechnik. Die erfindungsgemäße Referenzierung mithilfe der Ortungssonde ist ebenfalls apparativ unaufwendig und kostengünstig und im therapeutischen Ablauf zeitsparend, praktikabel und strahlenbelastungsfrei. Im Übrigen sind minimal-invasive Ortungssonden wie Katheter und Endoskope nicht mit erheblichen Belastungen des Patienten verbunden.

Die aus dem Stand der Technik bekannte Röntgennavigation wird erfindungsgemäß also durch eine auf der Magnetortung basierende Navigation ersetzt. Dies bedeutet nicht notwendigerweise, dass die Erfindung nicht auch in Kombination mit Röntgenabbildungen vorteilhaft eingesetzt werden kann. Darauf wird noch näher eingegangen. In solchen Fällen dient die Röntgenabbildung aber lediglich zur anfänglichen und damit vorzugsweise nur einmaligen Abbildung zu Referenzierungszwecken zusammen mit der Ortungssonde. Die laufende Überwachung und insbesondere die Berücksichtigung von Patientenbewegungen erfolgen röntgenunabhängig. In bestimmten Ausgestaltungen der Erfindung kann auch ganz auf die Röntgentechnik verzichtet werden, was den apparativen Aufwand erheblich reduziert und den Ablauf für das Klinikpersonal deutlich vereinfacht. Schließlich gibt es Ausführungsformen der Erfindung, bei denen zwar auch Röntgenabbildungen eingesetzt werden, diese aber insoweit vereinfacht werden, als nur noch Röntgenabbildungen mit einer einzelnen Abbildungsrichtung verwendet werden und aufwendige Verstellmechanismen entfallen können.

Die Erfindung bietet also eine effektive, einfache und ökonomische Druckwellennavigation mit geringer Patientenbelastung.

Bei einer Ausgestaltung der Erfindung ist die Ortungssonde ein Endoskop und weist damit eine optische Sichteinrichtung auf. Man kann also visuell verfolgen, wie sich das Endoskop, insbesondere die Endoskopspitze, der zu behandelnden Region nähert. Das Endoskop kann beispielsweise in den Harnleiter eingeführt und soweit vorgeschoben werden, dass ein zu behandelnder Nierenstein in dem Patienten sichtbar wird. Durch die infolge der Sichtkontrolle mögliche Führung des Endoskops kann eine räumliche Nähe zwischen Endoskop und Stein bzw. zu behandelnder Region hergestellt werden. Im einfachsten Fall kann man das Endoskop bis an den Stein heranbringen, sodass das Magnetortungselement, etwa eine Spule in der Endoskopspitze, direkt neben dem Stein positioniert ist. Wenn nun das Magnet-Tracking-System die Position des Magnetortungselements erfasst, kann es damit faktisch auch Positionen des Steins verfolgen, solange die direkte Nähe zwischen dem Magnetortungselement und dem Stein erhalten bleibt Letzteres kann über Sichtkontrolle sichergestellt werden. Röntgenabbildungen oder andere Abbildungen mit extrakorporalen bildgebenden Vorrichtungen sind nicht notwendig, können aber zur Erhöhung der Diagnosesicherheit anfänglich eingesetzt werden.

Je nach technischer Ausgestaltung des Endoskops und je nach genauer Art der Druckwellenbehandlung kann es problematisch sein, ein Endoskopteil im Fokusbereich zu belassen, während die Druckwellenbehandlung erfolgt. Beispielsweise können die Fokusbereiche von Stoßwellenlithotripsiegeräten klassische starre Endoskope beschädigen. Zumindest in solchen Fällen kommt eine andere Ausgestaltung der Erfindung in Betracht, bei der das Endoskop eine Abstandsmesseinrichtung enthält. Bevorzugt sind hier optische Abstandsmessverfahren, also solche mit Licht. Als Beispiele seien stereoskopische, holographische oder auch Laufzeitmessverfahren genannt, die eine quantitative Messung des Abstandes zwischen Endoskop, etwa Endoskopspitze, und beispielsweise einem Stein gestatten. Der gemessene Abstand kann dann rechnerisch bei der Navigation berücksichtigt werden, bildet also eine Korrektur des von dem Magnetortungsgerät erfassten Zielgebietes.

Eine weitere Variante verwendet einen Katheter in dem Messendoskop und führt das Endoskop, wie beschrieben, durch Sichtkontrolle in die Nähe der zu behandelnden Region. Die unmittelbare Nähe von Endoskop und zu behandelnder Region wird jedoch aus den genannten Gründen vermieden. Stattdessen wird ein das Magnetortungselement enthaltender Katheter aus dem Arbeitskanal des Endoskops heraus vorgeschoben und so positioniert, dass das Magnetortungselement selbst in unmittelbarer Näher der zu behandelnden Region angeordnet ist. Der Katheter kann gegenüber den Druckwellen unempfindlicher und/oder ein Einmalartikel sein. Die unmittelbare Nähe zwischen der zu behandelnden Region und dem Katheter oder auch, bei einer zuvor erläuterten Ausgestaltung, dem Endoskop kann natürlich neben einer Sichtkontrolle in der Schlussphase auch manuell-sensorisch erfolgen, etwa indem manuell das Anstoßen an einen Stein zu spüren ist.

Wie bereits erwähnt, kann die Erfindung durchaus in vorteilhafter Weise mit Röntgeneinrichtungen kombiniert werden. Das gilt auch allgemeiner für extrakorporale Abbildungstechniken, etwa Ultraschalldiagnostik.

Insbesondere kann eine solche Abbildung die per Sichtkontrolle, gegebenenfalls mit Abstandsmessung, erfolgte Annäherung zwischen Magnetortungselement und zu behandelnder Region verifizieren. Bei der Verwendung eines Katheters ohne Sichteinrichtung kann ein bildgebendes Verfahren die Sichtkontrolle ersetzen. Dann kann also der Katheter so angeordnet werden, dass sich in der Abbildung zeigt, dass er in einer unmittelbaren Nähe zur zu behandelnden Region liegt bzw. wie groß der verbleibende Abstand ist, um diesen als Korrektur zu berücksichtigen.

Von besonderem Interesse ist eine Kombination eines Katheters als Ortungssonde mit einer Abbildung in folgender Weise: Eine Röntgenabbildung oder andere extrakorporale Abbildung mit vergleichbarer Abbildungsachse dient zur Positionierung des Magnetortungselements in dem Katheter in einer Ebene senkrecht zu der Abbildungsachse. In der Richtung der Abbildungsachse selbst wird das Magnetortungselement nur mit dem Magnetortungsgerät lokalisiert, erfolgt aber keine gesonderte Kontrolle der Nähe zwischen zu behandelnder Region und Magnetortungselement. Dies ist vor allem dann gut vertretbar, wenn aus anderen Umständen, insbesondere wegen der anatomischen Rahmenbedingungen, ohnehin mit einer hinreichenden Genauigkeit auf diese Nähe in Richtung der Abbildungsachse geschlossen werden kann. Zum einen haben nämlich typische Druckwellenbehandlungen einen länglichen Fokusbereich, dessen Längserstreckung parallel zur Hauptausbreitungsrichtung der Druckwellen liegt. Zum anderen können die anatomischen Rahmenbedingungen bereits eine genauere Lokalisierung als die Ausdehnung dieses Fokusbereichs vorgeben. Die Positionierung durch die extrakorporale Abbildung soll dabei möglichst unter einem kleinen Winkel zu dieser Druckwellenausbreitungsrichtung vorgenommen werden, sodass die mit geringerer Genauigkeit erfasste Positionierung des Magnetortungselements relativ zu der zu behandelnden Region eine Richtung betrifft, in der der Fokusbereich relativ lang ist.

Bevorzugt ist dabei natürlich eine im Wesentlichen koaxiale Geometrie, beispielsweise mit mit der Hauptachse der Druckwellenausbreitung weitgehend übereinstimmender Röntgenachse. Insbesondere sind Druckwellenquellen bevorzugt, die eine Hohlgeometrie aufweisen, etwa eine Hohlspule einer Stoßwellenquelle. Dann kann die Röntgenabbildung durch den Hohlraum hindurch erfolgen und damit im Wesentlichen die Ebene mit der geringsten Fokusbereichausdehnung abbilden. Natürlich kann dabei insoweit eine leichte Verkippung zwischen den Achsen auftreten, als die in der Röntgenabbildung oder allgemeiner extrakorporalen Abbildung erfasste zu behandelnde Region im Bild nicht präzise mittig liegen muss. Die Abweichungen von der Mitte können dann durch entsprechende Einstellung der Druckwellenvorrichtung bzw. Stoßwellenvorrichtung berücksichtigt werden, ohne dass die Röntgenapparatur nachgestellt werden müsste. Im Rahmen des Abbildungsbereichs können also durchaus Achsverkippungen auftreten. Bei dieser Ausgestaltung der Erfindung können die Achsen jedenfalls auch koaxial angeordnet werden.

Im Prinzip kann natürlich auch die Sichtkontrolle, gegebenenfalls in Kombination mit der beschriebenen Abstandsmessung, zur Positionierung in der (Röntgen-) Abbildungsrichtung selbst dienen, also in der Richtung, die mit einer einzigen Röntgenabbildung nicht sichtbar ist. Natürlich kann die Sichtkontrolle dabei auch zum Einführen eines Endoskops in den relevanten Bereich dienen. Hier könnte die mit der extrakorporalen Abbildung erzielbare zusätzliche Sicherheit und Genauigkeit kombiniert werden mit dem Verzicht auf eine Vielzahl von Röntgenabbildungen.

Wenn eine Röntgeneinrichtung zur Verfügung steht, die verschiedene Abbildungsrichtungen zulässt, kann die Ortungssonde, insbesondere im Fall eines einfachen Katheters ohne Sichteinrichtung, aber auch mit mindestens zwei Röntgenabbildungen dreidimensional im Körper positioniert werden. Bei dieser Positionierung kann ein eventueller Restabstand zu der zu behandelnden Region durch die Abbildung gemessen werden. In der Regel entfällt eine solche Messung, weil der Katheter ohnehin in die direkte Nähe gebracht werden kann. Die Erfindung bietet auch hier noch Vorteile, nämlich die Verwendbarkeit des Magnet-Tracking-Systems für die weitere Navigation, sodass nur anfänglich geröntgt werden muss.

Bei den verschiedenen Kombinationen mit extrakorporalen Abbildungstechniken muss der Katheter für die verwendete extrakorporale Abbildungstechnik sichtbar sein. Beispielsweise können Einsätze (Marker) mit erhöhter Röntgenabsorption Verwendung finden. Auch das Magnetortungselement selbst kann diese Funktion gegebenenfalls erfüllen. Endoskope sind in der Regel ohnehin recht gut sichtbar, jedenfalls wenn es sich um starre Metallkonstruktionen handelt. Es sind aber auch flexible und röntgenkontrastarme Ortungssonden mit Sichteinrichtung etwa über ein flexibles Glasfaserbündel denkbar, die nach der hier verwendeten Abgrenzung als Endoskop zu bezeichnen sind. Auch hier ist durch das Magnetortungselement oder einen anderen Marker für einen ausreichenden Kontrast im Röntgenbild zu sorgen.

Die Druckwellenquelle ist extrakorporal angeordnet. Sie kann über ein Flüssigkeitsvolumen, etwa in einem Balg, an den Körper angekoppelt werden. Von besonderer Bedeutung ist die Erfindung dabei für Stoßwellenlithotripter.

Im Folgenden wird die Erfindung anhand eines Ausführungsbeispiels näher erläutert, wobei die einzelnen Merkmale auch in anderen Kombinationen erfindungswesentlich sein können und sich, wie bereits erwähnt, implizit auf alle Kategorien der Erfindung beziehen.
- Figur 1: zeigt eine schematische Darstellung einer erfindungsgemäßen Vorrich- tung mit einem Schnitt durch einen menschlichen Unterleib.
- Figur 2: zeigt eine ähnliche Darstellung wie Figur 1, aber ein zweites Ausführungs- beispiel.
- Figur 3: zeigt eine ähnliche Darstellung wie Figur 1, aber ein drittes Ausführungs- beispiel.

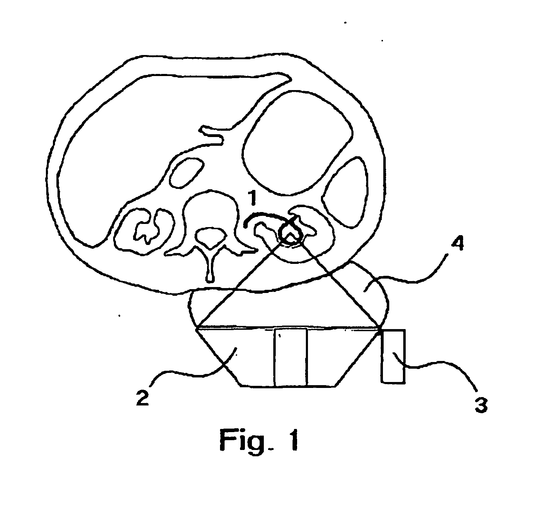

Figur 1 zeigt im oberen Bereich einen schematischen Schnitt durch den Unterleib eines Menschen. Man erkennt im unteren Bereich mittig die Wirbelsäule und links und rechts davon jeweils eine Niere. Die in der Figur rechte Niere enthält einen Katheter 1 mit der sogenannten Doppel-J-Form. Der Katheter 1 enthält in seinem vordersten dargestellten Bereich eine oder auch mehrere metallische Magnetspulen, die einerseits als Magnetortungselement dienen und andererseits einen guten Röntgenkontrast bieten.

Speziell bei urologischen Steinen ist die Einführung eines Katheters ohnehin von Vorteil, um Verstopfungen der ableitenden Gefäße durch Steinbruchstücke zu vermeiden bzw. durch den Katheter hindurch eine Harnableitung unabhängig von dem Transportweg der Steinbruchstücke zur gewährleisten.

Ein rechts unten eingezeichnetes Magnetortungsgerät 3 kann die Position der Magnetspulen erfassen. Es ist in diesem Beispiel fest montiert an einem an sich bekannten Stoßwellengerät 2 mit integrierter Fokussiereinrichtung, das über einen flüssigkeitsgefüllten Faltenbalg 4 an den Körper angekoppelt ist. Die von dem Stoßwellengerät 2 durch das Flüssigkeitsvolumen in dem Faltenbalg 4 auf einen Punkt in der Schlaufe des Katheters 1 zusammenlaufenden Linien symbolisieren die fokussierten Stoßwellen aus der Quelle 2. Mit ihnen soll ein Nierenstein zertrümmert werden, der in dem angedeuteten Fokusbereich liegt.

Der Katheter 1 wird zunächst über den Harnleiter eingeführt und in an sich bekannter Weise in den relevanten Bereich der Niere gebracht. Dort kann er über eine an sich konventionelle Folge von Röntgenbildern positioniert bzw. in seiner Position verifiziert werden. Hier sind verschiedene bekannte Verfahren der Fluoroskopie oder Radiographie geeignet. Letztlich ist dann aus den Röntgenaufnahmen bekannt, dass der Katheter 1 mit den Magnetspulen in unmittelbarer Nähe des Steins angeordnet ist. Die Magnetspulen dienen also zur Markierung der Steinposition, und zwar sowohl in der Röntgenabbildung als auch im Magnet-Tracking.

Da das Magnetortungsgerät 3 und das Stoßwellengerät 2 in einer festen räumlichen Beziehung zueinander montiert sind, können die Positionsdaten des Magnet-Tracking-Systems unter Berücksichtigung dieser räumlichen Beziehung direkt als Zielkoordinatendaten für das Stoßwellengerät 2 Verwendung finden. Somit lässt sich also auch der Stoßwellenfokus auf den Stein einstellen, etwa indem eine Baueinheit, an der das Stoßwellengerät 2 und das Magnetortungsgerät 3 montiert sind, verdreht oder verschoben wird. Gleichzeitig kann die räumliche Beziehung zwischen Stoßwellenfokus und Magnetspulenpositionen numerisch und oder grafisch angezeigt werden. Wenn eine hinreichend genaue Übereinstimmung hergestellt ist, kann die eigentliche Stoßwellenbehandlung beginnen.

Während dieser Behandlung verfolgt das Magnet-Tracking-System 3 die Lage der Magnetspulen beständig weiter. Sollte sich der Patient bewegen oder infolge anderer Umstände eine Änderung der Magnetspulenpositionen auftreten, kann die Behandlung unterbrochen und nach Neujustage fortgesetzt werden.

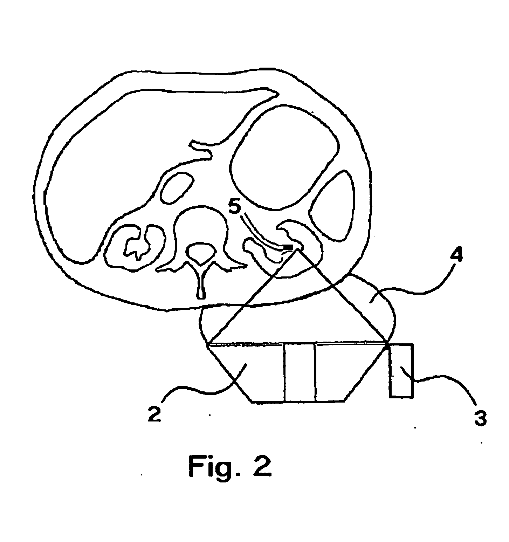

Das zweite Ausführungsbeispiel in Figur 2 unterscheidet sich von dem ersten aus Figur 1 zunächst hinsichtlich der Ortungssonde. Hier ist ein Endoskop 5 so eingeführt, dass die in der Figur dunkel eingezeichnete Endoskopspitze in der Nähe des Fokusbereichs des Stoßwellengerätes 2 liegt. Das Endoskop ist eine starr rohrförmige Konstruktion mit einer optischen Sichteinrichtung für die Bedienungsperson. Dabei kann der Einführvorgang bereits unter optischer Sichtkontrolle erfolgen, etwa indem an Gefäßeingängen oder unter Zuhilfenahme der abgebogenen Endoskopspitze der richtige Weg gewählt wird. Insbesondere kann in der Niere mithilfe der optischen Sichtkontrolle eine Anordnung der Endoskopspitze in Steinnähe erfolgen.

Das Endoskop enthält an seiner Spitze eine in der Figur nicht näher dargestellte optische Abstandsmesseinrichtung, beispielsweise über Laufzeitmessungen von ausgestrahltem und am Stein reflektiertem Licht.

Das Magnet-Tracking-System 3 erlaubt wie beim ersten Ausführungsbeispiel die Bestimmung der Position einer Magnetspule in der Endoskopspitze. Unter Berücksichtigung des gemessenen Restabstandes zum Stein kann dann der Fokusbereich des Stoßwellengerätes 2 richtig eingestellt werden. Hierbei ist zu erwähnen, dass Magnet-Tracking-Systeme nicht nur dreidimensionale Ortsangaben sondern auch Richtungsangaben liefern können und damit auch die Richtung, in der der Abstand durch das Abstandsmessgerät gemessen wurde, grundsätzlich bekannt sein kann. Man kann hier aber auch von Näherungen ausgehen, wenn es sich um geringe Restabstände handelt.

Ferner kann das Endoskop 5 einen Katheter enthalten, der mit der darin integrierten Magnetspule direkt bis zum Stein vorgeschoben wird. In diesem Fall kann die Abstandsmesseinrichtung entfallen.

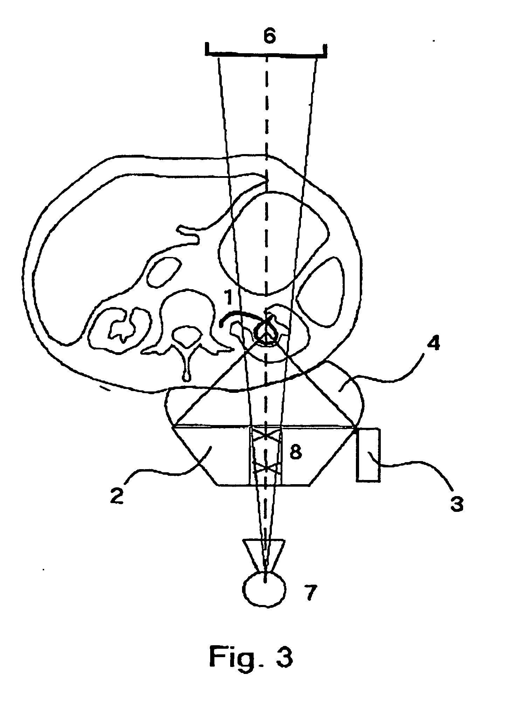

Bei dem dritten Ausführungsbeispiel aus Figur 3 ist das Beispiel vorteilhaft mit einer Röntgenabbildung in einer Richtung aus Figur 1 kombiniert. Dabei lässt sich die Tatsache nutzen, dass der Fokusbereich des Stoßwellengerätes 2 eine längliche Form hat, also bezogen auf die figürliche Darstellung vertikal länger ist als horizontal und senkrecht zur Zeichenebene. Es ist also vor allem erstrebenswert, die Position des Steins bzw. der Magnetspule in einer Ebene zu bestimmen, die möglichst wenig aus einer senkrecht auf der Zeichenebene stehenden und diese horizontal schneidenden Ebene verkippt ist. Besonders vorteilhaft lässt sich dabei eine zum Stoßwellengerät 2 koaxiale Röntgenapparatur einsetzen, die in Figur 3 zusätzlich zu Figur 1 gezeigt ist.

Das Stoßwellengerät 2, insbesondere die Quelle, befindet sich im bzw. ist um den Strahlengang des Röntgensystems 6, 7 herum angeordnet. Das Röntgensystem ist nur schematisch dargestellt und weist eine Röntgenquelle 7 auf der körperfernen Seite des Stoßwellengerätes 2 und einen Bildverstärker 6 auf der zu dem Stoßwellengerät 2 entgegengesetzten Seite des Körpers auf. Das Stoßwellengerät 2 hat eine Hohlgeometrie mit einem zentral axialen Durchtritt. Dies wird durch eine Hohlspulenkonstruktion der Quelle erreicht. In dem Hohldurchtritt sind in der Figur schematisch angedeutete und mit 8 bezeichnete fadenkreuzartige röntgenabsorbierende Markierungen angebracht. Damit kann der Gesamtaufbau aus Stoßwellengerät 2, Röntengerät 6, 7 und Magnetortungsgerät 3 so ausgerichtet werden, dass der Stein und die Magnetspule im Fadenkreuz bzw. nahe genug daran liegen. Alternativ dazu kann es auch genügen, den Stein überhaupt im Röntgenbild lokalisieren zu können, um dann das Stoßwellengerät entsprechend auszurichten. Hierbei muss die Röntgeneinrichtung nicht mit bewegt werden.

Diese Ausgestaltung der Erfindung kommt vor allem für Indikationen in Betracht, bei denen sich schon aus den anatomischen Gegebenheiten eine bestimmte Position der zu behandelnden Region, hier des Steins, und des Katheters ergeben. Beispielsweise können im Nierenbecken oder am Ausgang eines in das Nierenbecken mündenden Nierenkelchs angeordnete Steine vor allem in der Längsrichtung des länglich geformten Nierenbeckens eine größere Positionsvarianz haben. In der Regel wird dabei aber annähernd quer zu dieser Längsrichtung behandelt. Wenn hier also sichergestellt ist, dass der Stein und die Magnetspule in dem Katheter in einer in der genannten Richtung angefertigten Röntgenaufnahme richtig liegen, so kann aufgrund der anatomischen Gegebenheiten auf eine Positionierung in der "Tiefenrichtung" verzichtet werden. Das Nierenbecken hat nämlich in dieser Richtung Ausdehnungen in der Größenordnung von 2 cm, wohingegen eine typische Fokusbereichlänge eines Stoßwellengerätes in der Größenordnung von 4 cm liegt. Hier ist also eine zweidimensionale Ausrichtung durch das Röntgenbild ausreichend. Bei der Einstellung der Fokustiefe kann dann unterstellt werden, dass die Magnetspule nahe genug beim Stein liegt, also ohne zusätzliche Referenzierung mit dem Magnet-Tracking-System gearbeitet werden kann. Durch die Erfassung der Position des Magnetortungselements in der Abbildungsrichtung des ersten Röntgenbildes kann man sich hier also ein zweites Röntgenbild mit deutlich geänderter Abbildungsrichtung sparen. Dies reduziert nicht nur die Röntgenbelastung des Patienten sondern vereinfacht auch die Röntgenapparatur, weil auf Schwenkmechanismen verzichtet werden kann.

Übrigens bietet sich eine Kombination mit einer Ultraschallabbildung gerade für die erwähnte Hohlgeometrie des Stoßwellengeräts 2 an. Der Ultraschallkopf kann dann durch den Hohlraum geführt werden und alternativ oder zusätzlich zur Röntgenabbildung extrakorporale Abbildungen erzeugen.

Im Prinzip sind auch Kombinationen von Röntgenabbildungen mit endoskopischen Sichtkontrollen denkbar, insbesondere wenn in der gerade beschriebenen Weise die eine Röntgenabbildung in nur einer Abbildungsrichtung als zusätzliche Sicherheit oder zur Erhöhung der Genauigkeit einer Sichtkontrolle gewünscht ist und diese aber für die dritte Richtung als ausreichend angesehen wird. In vielen praktischen Fällen wird aber die Sichtkontrolle alleine bereits ausreichen, sodass ganz auf Röntgentechniken verzichtet werden kann.

## Patentansprüche

1. Vorrichtung zur Behandlung des menschlichen oder tierischen Körpers mit fokussierten Druckwellen,
welche Vorrichtung aufweist:
- eine extra-korporale Druckwellenquelle (2) zur Erzeugung der Druckwellen mit einer integrierten Fokussiereinrichtung (2) zum Fokussieren der Druckwellen auf einen Fokusbereich in dem zu behandelnden Körper,
- eine Ortungssonde (1, 5) zum Einführen in den zu behandelnden Körper in der Nähe der zu behandelnden Region,
**gekennzeichnet durch**
- ein Magnetortungselement in Form einer aktiven oder passiven Spule in der Ortungssonde (1, 5) und
- ein Magnetortungsgerät (3) zur Ortung des Magnetortungselements in der Ortungssonde (1, 5) in dem zu behandelnden Körper und damit zur Navigation bei der Behandlung.

2. Vorrichtung nach Anspruch 1, bei der die Ortungssonde (1, 5) ein Endoskop (5) mit einer optischen Sichteinrichtung ist.

3. Vorrichtung nach Anspruch 2, bei der das Endoskop (5) eine Abstandsmesseinrichtung zur Ermittlung des Abstandes zwischen dem Endoskop (5) und einer zu behandelnden Region des Körpers aufweist.

4. Vorrichtung nach Anspruch 3, bei der die Abstandsmesseinrichtung eine optische Abstandsmesseinrichtung ist.

5. Vorrichtung nach einem der Ansprüche 2 - 4, bei der das Endoskop (5) einen Katheter enthält und das Magnetortungselement in dem Katheter angeordnet ist.

6. Vorrichtung nach einem der vorstehenden Ansprüche mit einer extrakorporalen Abbildungseinrichtung (6 - 8), bei der die Ortungssonde (1, 5) mit der extrakorporalen Abbildungseinrichtung (6 - 8) abbildbar ist.

7. Vorrichtung nach Anspruch 6, bei der die extrakorporale Abbildungseinrichtung (6 - 8) eine Röntgeneinrichtung (6 - 8) ist.

8. Vorrichtung nach Anspruch 6 oder 7, bei der die extrakorporale Abbildungseinrichtung (6 - 8) koaxial zu der Druckwellenquelle (2) angeordnet werden kann.

9. Vorrichtung nach einem der Ansprüche 6 - 8, bei der die Ortungssonde (1, 5) ein Katheter (1) ist.

10. Vorrichtung nach einem der vorstehenden Ansprüche, bei der die Druckwellenquelle (2) zur Ankopplung an den Körper über ein Flüssigkeitsvolumen (4) ausgelegt ist.

11. Vorrichtung nach Anspruch 10, bei der die Druckwellenquelle (2) eine Stoßwellenquelle und die Vorrichtung ein Stoßwellenlithotripter ist.

## Claims

1. An apparatus for treating a human or animal body with focused pressure waves,
said apparatus comprising:
- an extracorporeal pressure wave source (2) for a generation of said pressure waves, with an integrated focusing device (2) for focusing said pressure waves onto a focus region in said body to be treated,
- a locating probe (1, 5) for an insertion into said body to be treated near said region to be treated,
**characterized by**
- a magnetic locating unit in form of an active or passive coil in said locating probe (1, 5) and
- a magnetic locating device (3) for locating said magnetic locating unit in said locating probe (1, 5) in said body to be treated and thereby for navigating during said treatment.

2. The apparatus according to claim 1, wherein said locating probe (1, 5) is an endoscope (5) with an optical vision device.

3. The apparatus according to claim 2, wherein said endoscope (5) comprises a distance meter device for determining the distance between said endoscope (5) and a region to be treated of said body.

4. The apparatus according to claim 3, wherein said distance meter device is an optical distance meter device.

5. The apparatus according to one of claims 2 - 4, wherein said endoscope (5) contains a catheter and said magnetic locating unit is arranged in said catheter.

6. The apparatus according to one of the preceding claims having an extracorporeal imaging device (6 - 8), wherein said locating probe (1, 5) can be imaged with said imaging device (6 - 8).

7. The apparatus according to claim 6, wherein said extracorporeal imaging device (6 - 8) is an X-ray device (6 - 8).

8. The apparatus according to claim 6 or 7, wherein said extracorporeal imaging device (6 - 8) can be arranged coaxially to said pressure wave source (2).

9. The apparatus according to one of claims 6 - 8, wherein said locating probe (1. 5) is a catheter (1).

10. The apparatus according to one of the preceding claims, wherein said pressure wave source (2) is adapted for being coupled to said body via a volume of liquid (4).

11. The apparatus according to claim 10, wherein said pressure wave source (2) is a shock wave source and said apparatus is a shock wave lithotripter.

## Revendications

1. Dispositif pour le traitement du corps humain ou animal avec des ondes de pression focalisées, lequel dispositif comprenant :
- une source d'ondes de pression (2) extracorporelle pour produire les ondes de pression avec un dispositif de focalisation (2) intégré pour focaliser les ondes de pression sur une zone de foyer dans le corps à traiter,
- une sonde de localisation (1, 5) destinée à être introduite dans le corps à traiter dans le voisinage de la zone à traiter,
**caractérisé par**
- un élément de localisation magnétique sous forme d'une bobine active ou passive dans la sonde de localisation (1, 5) et
- un appareil de localisation magnétique (3) pour localiser l'élément de localisation magnétique dans la sonde de localisation (1, 5) dans le corps à traiter et pour naviguer ainsi pendant le traitement.

2. Dispositif selon la revendication 1, dans lequel la sonde de localisation (1, 5) est un endoscope (5) avec un dispositif de vue optique.

3. Dispositif selon la revendication 2, dans lequel l'endoscope (5) présente un dispositif de mesure de distance pour déterminer la distance entre l'endoscope (5) et une région à traiter du corps.

4. Dispositif selon la revendication 3, dans lequel le dispositif de mesure de distance est un dispositif de mesure de distance optique.

5. Dispositif selon l'une des revendications 2 à 4, dans lequel l'endoscope (5) contient un cathéter et l'élément de localisation magnétique est placé dans le cathéter.

6. Dispositif selon l'une des revendications précédentes comprenant un dispositif de représentation extracorporel (6 - 8) avec lequel la sonde de localisation (1, 5) peut être représentée avec le dispositif de représentation extracorporel (6 - 8).

7. Dispositif selon la revendication 6, dans lequel le dispositif de représentation extracorporel (6 - 8) est un dispositif de rayons X (6 - 8).

8. Dispositif selon la revendication 6 ou 7, dans lequel le dispositif de représentation extracorporel (6 - 8) peut être placé de manière coaxiale à la source d'ondes de pression (2).

9. Dispositif selon l'une des revendications 6 à 8, dans lequel la sonde de localisation (1, 5) est un cathéter (1).

10. Dispositif selon l'une des revendications précédentes dans lequel la source d'ondes de pression (2) est conçue pour s'accoupler au corps par un volume de liquide (4).

11. Dispositif selon la revendication 10, dans lequel la source d'ondes de pression (2) est une source d'ondes de choc et le dispositif est un lithotripteur par ondes de choc.
